Europäisches Patentamt

(19) European Patent Office

Office européen des brevets

(11) **EP 0 930 071 A1**

(12) **DEMANDE DE BREVET EUROPEEN**

(43) Date de publication:
21.07.1999 Bulletin 1999/29

(51) Int. Cl.$^6$: **A61K 31/70**

(21) Numéro de dépôt: 98403241.7

(22) Date de dépôt: 21.12.1998

(84) Etats contractants désignés:
AT BE CH CY DE DK ES FI FR GB GR IE IT LI LU
MC NL PT SE
Etats d'extension désignés:
AL LT LV MK RO SI

(30) Priorité: 29.12.1997 FR 9716639

(71) Demandeur: **Laboratoire Chauvin**
34009 Montpellier Cédex 1 (FR)

(72) Inventeurs:
• Coquelet, Claude
34470 Perols (FR)
• Latour, Elisabeth
Montpellier (FR)

(74) Mandataire: **Le Guen, Gérard et al**
**CABINET LAVOIX**
**2, place d'Estienne d'Orves**
**75441 Paris Cédex 09 (FR)**

(54) **Utilisation de la flavine-adénine-dinucléotide pour la préparation de compositions ophtalmiques utiles pour le traitement de l'oeil sec**

(57) La présente invention concerne l'utilisation de la flavine-adénine-dinucléotide (FAD) ou de l'un de ses sels avec une base ou un acide pharmacologiquement acceptable pour la préparation d'une composition ophtalmique topique destinée au traitement de l'oeil sec.

EP 0 930 071 A1

## Description

La présente invention concerne une composition ophtalmique topique utile pour le traitement de l'oeil sec et notamment pour augmenter la sécrétion lacrymale.

La pathologie de l'oeil sec est aujourd'hui une pathologie fréquente.

On peut considérer que quatre troubles physiologiques principaux sont déterminants dans la manifestation et l'évolution de cette pathologie. Ces troubles consistent en une diminution de la production des larmes, une diminution de la densité des cellules à mucus conjonctivales et du glycogène cornéen, une augmentation de la desquamation épithéliale cornéenne et une déstabilisation de l'interface larmes-cornée.

Les méthodes de traitement de la pathologie de l'oeil sec actuellement disponibles peuvent être classées selon trois catégories. Elles visent soit à suppléer au déficit de larmes par des larmes artificielles ou gels lacrymaux, soit à conserver les larmes existantes en évitant leur drainage avec des clous lacrymaux soit à stimuler la production de larmes avec des agents tels que la Pilocarpine, médicament approuvé au Canada en 1997 et administré par voie orale.

De ces trois types de traitement, celui mettant en oeuvre des molécules capables d'agir sur les glandes lacrymales et donc de stimuler pharmacologiquement la sécrétion de la phase aqueuse du film lacrymal est sans contexte le plus intéressant sur le plan thérapeutique. Il permet de suppléer non seulement au déficit en eau mais également au déficit en constituants électrolytiques et protéiques nécessaires au bon fonctionnement de l'épithélium cornéo-conjonctival.

La Pilocarpine, est actuellement, dans les modèles animaux, la molécule sécrétagogue de référence. Elle stimule efficacement la sécrétion de la glande lacrymale quelle que soit la voie d'administration. Toutefois, elle induit simultanément des effets secondaires indésirables.

La présente invention a précisément pour objet de proposer l'utilisation d'un nouvel agent capable de stimuler la sécrétion lacrymale.

Ce composé dérive d'une molécule biologique : la riboflavine.

Plus précisément, il s'agit de la flavine-adénine-dinucléotide ou de l'un de ses sels avec une base ou un acide pharmacologiquement acceptable, encore designée ci-après sous le sigle FAD.

La FAD est une molécule déjà proposée dans le domaine ophtalmique, pour le traitement des inflammations cornéennes, liées en particulier à la carence en vitamine B2.

De manière inattendue, la demanderesse a mis en évidence la capacité de cette molécule à augmenter significativement le volume lacrymal.

En conséquence, la présente invention a pour objet l'utilisation de la flavine-adénine-dinucléotide ou de l'un de ses sels avec une base ou un acide pharmacologiquement acceptable pour la préparation d'une composition ophtalmique topique destinée au traitement de l'oeil sec.

Plus particulièrement, la présente invention a pour objet l'utilisation de la flavine-adénine-dinucléotide ou de l'un de ses sels avec une base ou un acide pharmacologiquement acceptable pour la préparation d'une composition ophtalmique topique destinée à stimuler la sécrétion lacrymale.

La FAD représente une alternative intéressante à la Pilocarpine, molécule sécrétagogue de référence, à la fois sur la plan efficacité et sur le plan toxicologique.

Tout d'abord, la FAD administrée par voie oculaire, démontre chez le lapin des effets stimulants comparables à ceux de la Pilocarpine et ce pour des doses plus réduites.

C'est ainsi que la FAD en instillation unique en solution aqueuse à 1 %, augmente le volume lacrymal d'un facteur 2 environ, 20 minutes après l'administration, comme cela est observé avec une solution de Pilocarpine à 2 %. Cette augmentation du volume lacrymal par la FAD est de l'ordre de 116 %.

Une étude plus approfondie montre par ailleurs que la FAD en solution aqueuse présente, dans le modèle de stimulation de la production de larmes chez le lapin un effet-dose et un effet-temps après administration topique oculaire unique : on ne note pas d'effet à une dose de 0,01%. On observe une augmentation d'environ deux fois le volume lacrymal à une dose de 1 %, 20 minutes après l'instillation, et une valeur intermédiaire pour la dose à 0,1 %.

Comme pour une solution de Pilocarpine à 2 %, l'optimum d'activité est observé entre 10 et 20 minutes après l'instillation et la durée d'action est d'environ 40 minutes pour une solution de FAD à 0,1 %.

Par ailleurs, la FAD ne manifeste ne perd pas ses effets stimulants après administrations répétées. Après 6 jours de traitement avec une solution ophtalmique à 1 % chez le lapin, à raison de deux instillations par jour, la stimulation de la sécrétion de la phase aqueuse du film lacrymal demeure avantageusement identique à celle observée après administration unique.

Sur le plan toxicologique, la FAD, contrairement à la Pilocarpine, ne présente pas d'effets secondaires en administration topique oculaire. En fait, la FAD, existante dans le corps humain, est une des formes actives de la vitamine B2 et présente donc à ce titre un caractère d'innocuité.

Plus préférentiellement, la FAD est utilisée sous la forme de son sel disodique.

La composition ophtalmique topique incorporant la FAD peut être formulée sous la forme d'un gel, d'une pommade, d'un collyre ou d'un insert oculaire.

De préférence, la composition ophtalmique préparée selon l'invention est un collyre. Il s'agit préférentiellement d'un collyre prêt à l'emploi. La FAD y est formulée en solution aqueuse.

La FAD est utilisée dans le collyre préparé selon l'invention à raison d'environ 0,05 à 10 % en poids de la composition ophtalmique. De préférence, elle est mise en oeuvre à raison d'environ 0,1 % à 5 % en poids et plus préférentiellement entre environ 0,5 % et 2 %.

Le collyre préparé selon l'invention a par ailleurs le net avantage de se présenter sous une forme liquide et de faible viscosité comparativement aux formulations classiques proposées pour le traitement de l'oeil sec et qui se présentent sous la forme de gels ou de solutions très visqueuses. De part sa présentation liquide et très fluide, le collyre selon l'invention n'induit donc pas de troubles visuels lors de son instillation.

Le pH du collyre est avantageusement compris entre environ 5,0 et 8,0 et de préférence entre 5,5 et 7,0. Si nécessaire, il peut être ajusté avec de l'hydroxyde de sodium.

Le collyre préparé selon l'invention peut en outre contenir des adjuvants classiques des collyres tels que des agents conservateurs antimicrobiens et/ou des isotonisants.

A titre d'exemples de conservateurs antimicrobiens, susceptibles d'être présents, dans le collyre, on peut notamment citer le chlorure de benzalkonium, le mercurothiolate sodique, le chlorobutanol le thimerosal, le méthylparabène, le propylparabène, l'édetate de sodium et l'acide sorbique. Ces conservateurs antimicrobiens peuvent être utilisés à une quantité variant entre 0,001 % à 1 % en poids.

En ce qui concerne les agents appropriés pour ajuster la tonicité ou l'osmolarité du collyre on peut notamment citer le mannitol, le dextrose, la glycérine, le propylène glycol et les sels minéraux comme le chlorure de sodium. Lorsqu'ils sont utilisés, ces agents sont employés à des quantités comprises entre 0,1 et 10 % en poids.

Les exemples figurants ci-après sont présents à titre illustratif et non limitatif de la présente invention.

## MATERIEL ET METHODES

Les expériences sont réalisées sur des lapins mâles albinos néozélandais pesant de 2,4 kg à 3,8 kg, provenant de l'élevage Charles River France (St Aubin les Elbeuf - 76410 CLEON) et acclimatés pendant 5 jours minimum dans l'animalerie avant le début des expériences (température : $19 \pm 2$ °C ; humidité relative : $55 \pm 10$ %; éclairage : 12 heures d'éclairage artificiel - 12 heures de nuit).

### Produits et matériel

La fluorescéine et le chlorhydrate de bupivacaïne sont des produits SIGMA (St Quentin - Fallavier). Le chlorhydrate de bupivacaïne a été dissous dans du tampon phosphate de Dulbecco sans calcium ni magnésium (SEROMED).

Les anesthésiques (ROMPUN® : xylazine et IMALGENE® 1000 : kétamine) proviennent de chez COVELY (Genay).

Les microcapillaires "Microcaps" DRUMMOND de 1 µl sont fournis par POLYLABO (Strasbourg).

L'appareil de mesure est un spectrofluorimètre KONTRON SFM . Les cuves utilisées sont des cuves en quartz à 5 faces d'un volume de 4 ml (POLYLABO).

## EXEMPLE 1

### Préparation d'un collyre selon l'invention et mesure de son efficacité.

On prépare un collyre témoin prêt à l'emploi contenant 2 % en poids de chlorhydrate de Pilocarpine. Son osmolarité est ajustée à -0,53 °C avec 0,44 g de chlorure de sodium. Le pH est de 4.

En ce qui concerne la formulation selon l'invention, elle comprend 1 % en poids du sel disodique de FAD (produit sigma) dans du sérum physiologique. Son osmolarité et pH sont respectivement de -0,61 °C et 5,8.

Une anesthésie locale préalable est induite dans l'oeil droit des animaux par administration oculaire de 25 µl d'une solution de bupivacaïne à 0,5 %. Cinq minutes plus tard, une goutte (25 µl) de la solution testée est instillée dans le cul-de-sac conjonctival inférieur de l'oeil anesthésié. Les paupières sont ensuite fermées manuellement une fois.

Pour chaque solution testée, un lot d'animaux recevant, dans les mêmes conditions, 25 µl de sérum physiologique a été inclus comme contrôle (dans le cas du collyre contenant la Pilocarpine, le lot contrôle a été traité par l'excipient correspondant).

Mesure du volume lacrymal

Le volume lacrymal est mesuré selon une technique de dilution d'une solution de fluorescéine, proche des méthodes utilisées par Mishima et al. et Göbbels et al. (Invest. Ophthalmol. 1996 ; 5 (3) : 264-276 et Graefe's Arch. Clin. Exp. Ophthalmol. 1991 ; 229 : 147-149) chez l'homme. Le principe général de cette méthode est le suivant : une solution de fluorescéine de concentration connue est instillée sous un volume le plus faible possible afin d'éviter le larmoiement réflexe puis immédiatement répartie de façon homogène dans le film lacrymal par clignements successifs. Un prélèvement de larmes est effectué tout de suite après ; la fluorescence de cet échantillon est déterminée par fluorimétrie. Le volume lacrymal est calculé selon l'équation :

$$V = \frac{Vs.Qi}{Qs} - Vi$$

V : volume lacrymal
Vs : volume du prélèvement de larmes
Qi : quantité de fluorescéine instillée
Qs : quantité de fluorescéine dans le prélèvement de larmes
Vi : volume de la solution de fluorescéine instillée

Dix minutes après instillation de la solution testée, les animaux sont anesthésiés par administration intramusculaire d'un mélange kétamine (35 mg/kg) - xylazine (5 mg/kg) afin de faciliter le prélèvement de larmes et de rendre la mesure du volume lacrymal plus fiable.

Dix minutes après induction de l'anesthésie générale, 1 $\mu$l d'une solution aqueuse de fluorescéine à 1 % est instillé au niveau du canthus externe à l'aide d'un microcapillaire. Les paupières sont fermées manuellement deux fois en évitant toute pression sur le globe oculaire. Cinq secondes après l'instillation de fluorescéine, 1 $\mu$l de larmes est prélevé par capillarité au niveau du ménisque inférieur en évitant tout contact du microcapillaire avec la paupière inférieure et la surface oculaire.

Le prélèvement de larmes est dilué dans 4 ml de tampon phosphate isotonique pH 7,4 ($NaH_2PO_4$ : 32 mM : $Na_2HPO_4$ : 104 mM).

La fluorescence de ces échantillons est mesurée à température ambiante au spectrofluorimètre (longueur d'onde d'excitation : 496 nm ; longueur d'onde d'émission : 506 nm) par rapport à celle d'une solution de référence contenant 1,6 $\mu$g de fluorescéine (2,8 $\mu$g dans le cas de l'étude des effets de la Pilocarpine) dans 4 ml de tampon phosphate (100 % de fluorescence). Le 0 a été réglé avec le tampon phosphate seul.

La quantité de fluorescéine contenue dans le prélèvement de larmes (Qs) est calculée à partir du pourcentage de fluorescence relatif de l'échantillon (x) selon la formule :

$$Qs\ (\mu g) = \frac{x(\%)x1,6\ \mu g}{100} \ (2,8\ \mu g\ pour\ l'étude\ de\ la\ Pilocarpine)$$

Le volume lacrymal exprimé en $\mu$l est calculé selon l'équation donnée précédemment dans laquelle :

Vi = 1 $\mu$l        Qi = 10 $\mu$g        Vs = 1 $\mu$l
Le volume lacrymal est exprimé sous forme de moyenne $\pm$ écart-type.
Les résultats sont présentés dans le tableau I ci-après :

TABLEAU I

| Effets de la Pilocarpine et du FAD sur le volume lacrymal chez le lapin | | | |
|---|---|---|---|
| Traitement | Volume lacrymal | Augmentation du volume lacrymal | |
| | ($\mu$l) | ($\mu$l) | (%) |
| Excipient | 3,32 ± 0,42 (8) | - | - |
| Pilocarpine 2 % | 6,87 ± 0,76* (8) | 3,55 | 107 |
| Sérum physiologique | 3,57 ± 0,27 (5) | - | - |
| FAD 1 % | 7,71 ± 3,43* (7) | 4,14 | 116 |

\* Significativement différent du lot contrôle
Nombre d'animaux entre parenthéses.

Le volume lacrymal du lapin est significativement augmenté d'un facteur 2 environ, 20 minutes après instillation de 25 $\mu$l d'un collyre contenant 2 % de chlorhydrate de Pilocarpine, comparativement au lot contrôle traité par l'excipient correspondant.

On note que la FAD induit également une augmentation significative du volume lacrymal après administration oculaire dans les mêmes conditions expérimentales (116 %). Les effets observés dans cette étude confirment les propriétés sécrétagogues de cette molécule.

## EXEMPLE 2

**Analyse de l'effet de FAD sur la sécrétion lacrymale en fonction de la dose instillée et au cours du temps.**

L'étude effet-dose est réalisée après administration unique des différentes solutions testées, 20 minutes avant la mesure du volume lacrymal.

Les résultats sont présentés dans le tableau II ci-après.

TABLEAU II

| Traitement | Volume lacrymal | Augmentation du volume lacrymal | |
|---|---|---|---|
| | ($\mu$l) | ($\mu$l) | (%) |
| Excipient | 3,32 ± 0,42 (8) | - | - |
| Pilocarpine 2 % | 6,87 ± 0,76* (8) | 3,55 | 107 |
| Sérum physiologique | 3,57 ± 0,27 (5) | - | - |
| FAD 1 % | 7,71 ± 3,43* (7) | 4,14 | 116 |
| Sérum physiologique | 3,69 ± 0,40 (6) | - | - |
| FAD 0,1 % | 5,32 ± 1,30* (7) | 1,63 | 44 |
| Sérum physiologique | 2,93 ± 0,77 (11) | - | - |
| FAD 0,01 % | 2,77 ± 0,67 (14) | 0 | 0 |

\* Significativement différent du lot contrôle
nombre d'animaux entre parenthéses.

La FAD induit une augmentation significative, dose-dépendante, du volume lacrymal. A concentration élevée (1 %), les effets sécrétagogues du FAD sont comparables à ceux de la Pilocarpine.

Dans le cas de l'étude cinétique, une goutte (25 μl) de solution contenant 0,1 % de produit testé est instillée à différents temps avant la mesure du volume lacrymal.

Les résultats sont présentés dans le tableau III ci-après.

En tableau IV, sont présentés à titre comparatif, les résultats obtenus avec un collyre à base de Pilocarpine à 2%.

TABLEAU III

| Volume lacrymal du lapin à différents temps après instillation unique d'une solution contenant 0,1 % de FAD | | | | |
|---|---|---|---|---|
| Temps | Traitement | Volume lacrymal | Augmentation du volume lacrymal | |
| (mn) | | (μl) | (μl) | (%) |
| 5 | Sérum physiologique | $4,44 \pm 1,11$ (6) | - | - |
| | FAD 0,1 % | $7,04 \pm 1,27^*$ (6) | 2,60 | 59 |
| 10 | Sérum physiologique | $3,34 \pm 0,50$ (7) | - | - |
| | FAD 0,1 % | $5,92 \pm 1,92^*$ (8) | 2,58 | 77 |
| 20 | Sérum physiologique | $3,12 \pm 0,40$ (7) | - | - |
| | FAD 0,1 % | $5,18 \pm 0,71^*$ (8) | 2,06 | 66 |
| 40 | Sérum physiologique | $3,52 \pm 0,68$ (6) | - | - |
| | FAD 0,1 % | $4,52 \pm 0,81^*$ (6) | 1,00 | 28 |
| 60 | Sérum physiologique | $3,10 \pm 0,46$ (7) | - | - |
| | FAD 0,1 % | $3,22 \pm 0,53$ (7) | 0,12 | 4 |

* Significativement différent du lot contrôle
nombre d'animaux entre parenthéses

TABLEAU IV

| Volume lacrymal du lapin à différents temps après instillation unique d'un collyre contenant 2% de chlorhydrate de Pilocarpine | | | | |
|---|---|---|---|---|
| Temps | Traitement | Volume lacrymal | Augmentation du volume lacrymal | |
| (mn) | | (μl) | (μl) | (%) |
| 10 | Excipient | $3,56 \pm 1,07$ (6) | - | - |
| | Pilocarpine 2% | $9,81 \pm 3,17^*$ (6) | 6,25 | 176 |
| 20 | Excipient | $3,32 \pm 0,42$ (8) | - | - |
| | Pilocarpine 2% | $6,87 \pm 0,76^*$ (8) | 3,55 | 107 |
| 40 | Excipient | $3,97 \pm 0,39$ (6) | - | - |
| | Pilocarpine 2% | $5,28 \pm 0,73^*$ (8) | 1,31 | 33 |
| 60 | Excipient | $3,59 \pm 1,04$ (6) | - | - |
| | Pilocarpine 2% | $4,55 \pm 1,15$ (7) | 0,96 | 27 |

* Significativement différent du lot contrôle
nombre d'animaux entre parenthéses.

On note que l'activité maximale du FAD se manifeste environ 10 minutes après instillation unique d'une solution à 0,1 %.

La durée d'action de ce produit administré en solution aqueuse sans additif viscosifiant ou gélifiant susceptible d'augmenter leur rémanence au niveau du site d'action, est de 40 minutes environ, similaire à celle du collyre de référence à base de Pilocarpine.

## EXEMPLE 3

### Etude du phénomène d'échappement.

Des solutions contenant 1 % de produit testé sont utilisées.

Le premier jour (J1), le volume lacrymal est déterminé 20 minutes après instillation d'une goutte, selon le protocole décrit en exemple 1.

Le traitement est ensuite administré de J2 à J7 sans anesthésie locale préalable, à raison de 2 instillations par jours espacées de 8 heures pendant la semaine (espacées de 5 heures le week-end).

Le huitième jour (J8), le volume lacrymal est mesuré selon le protocole décrit dans les mêmes conditions qu'à J1.

Les résultats obtenus avec le collyre témoin et le collyre selon l'invention figurent dans le tableau V.

TABLEAU V

| Traitement | J1 | | | J8 | | |
|---|---|---|---|---|---|---|
| | Volume lacrymal | Augmentation du volume lacrymal | | Volume lacrymal | Augmentation du volume lacrymal | |
| | ($\mu$l) | ($\mu$l) | (%) | ($\mu$l) | ($\mu$l) | (%) |
| Excipient | 2,91$\pm$0,60 (5) | - | - | 3,16$\pm$0,58 (6) | - | - |
| Pilocarpine 2% | 9,24$\pm$1,11*(6) | 6,33 | 218 | 7,90$\pm$1,86* (7) | 4,74 | 150 |
| Sérum physiologique | 308 $\pm$ 0,61 (8) | - | - | 3,36 $\pm$ 0,49 (8) | - | - |
| FAD 1 % | 6,83$\pm$3,44* (7) | 3,75 | 122 | 7,70 $\pm$ 3,93* (8) | 4,34 | 129 |

*Significativement différent du lot contrôle
nombre d'animaux entre parenthéses.

Aucun phénomène d'échappement n'est observé après administrations répétées de la FAD, à raison de 2 instillations par jour pendant 6 jours.

## EXEMPLE 4

Une étude de l'effet de la FAD à 10% sur le volume lacrymal chez le lapin montre, 20 minutes après l'instillation d'une goutte du collyre selon l'invention, une augmentation de 3,91 $\mu$l soit 147% par rapport à une solution de sérum physiologique.

**Revendications**

1.    Utilisation de la flavine-adénine-dinucléotide (FAD) ou de l'un de ses sels avec une base ou un acide pharmacologiquement acceptable pour la préparation d'une composition ophtalmique topique destinée au traitement de l'oeil sec.

2.    Utilisation de la flavine-adénine-dinucléotide ou de l'un de ses sels avec une base ou un acide pharmacologiquement acceptable pour la préparation d'une composition ophtalmique topique destinée à stimuler la sécrétion lacrymale.

3.    Utilisation selon la revendication 1 ou 2 caractérisée en ce que la FAD est utilisée sous la forme de son sel disodique.

4. Utilisation selon la revendication 1, 2 ou 3 caractérisée en ce que la composition ophtalmique topique se présente sous la forme d'un collyre.

5. Utilisation selon la revendication 4 caractérisée en ce que la FAD est présente dans la composition ophtalmique à raison d'environ 0,05 % à 10 % en poids de la composition ophtalmique.

6. Utilisation selon la revendication 5 caractérisée en ce que la FAD est présente à raison de 0,1 à 5 % en poids de la composition ophtalmique.

Office européen
des brevets

**RAPPORT DE RECHERCHE EUROPEENNE**

Numéro de la demande

EP 98 40 3241

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | PATENT ABSTRACTS OF JAPAN<br>vol. 096, no. 008, 30 août 1996<br>& JP 08 104636 A (SENJU PHARMACEUT CO LTD), 23 avril 1996<br>* abrégé * | 1-6 | A61K31/70 |
| X | DATABASE WPI<br>Section Ch, Week 9527<br>Derwent Publications Ltd., London, GB;<br>Class A96, AN 95-203736<br>XP002063116<br>& JP 07 118147 A (LION CORP), 9 mai 1995<br>* abrégé * | 1-6 | |
| X | DATABASE WPI<br>Section Ch, Week 8837<br>Derwent Publications Ltd., London, GB;<br>Class B02, AN 88-260488<br>XP002063117<br>& JP 63 188627 A (ROHTO PHARM CO LTD), 4 août 1988<br>* abrégé * | 1-6 | |
| | | | **DOMAINES TECHNIQUES RECHERCHES (Int.Cl.6)** |
| A | LEUS H.F.: "Vitamins and coenzymes in ophthalmology"<br>VOPR. MED. KHIM.,<br>vol. 38, no. 5, 1992, pages 53-57,<br>XP002063114<br>* le document en entier * | 1-6 | A61K |
| A | ONO ET AL.: "Determination of Vitamin B2 in Rat Lens"<br>VITAMIN,<br>vol. 67, no. 5-6, 1993, pages 243-247,<br>XP002063115<br>* abrégé * | 1-6 | |

-/--

Le présent rapport a été établi pour toutes les revendications

| Lieu de la recherche | Date d'achèvement de la recherche | Examinateur |
|---|---|---|
| LA HAYE | 23 mars 1999 | A. Jakobs |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Office européen
des brevets

**RAPPORT DE RECHERCHE EUROPEENNE**

Numéro de la demande

EP 98 40 3241

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | FR 2 715 303 A (BERQUE JEAN) 28 juillet 1995 * revendications 2,4 * ----- | 1-6 | |

DOMAINES TECHNIQUES RECHERCHES (Int.Cl.6)

Le présent rapport a été établi pour toutes les revendications

| Lieu de la recherche | Date d'achèvement de la recherche | Examinateur |
|---|---|---|
| LA HAYE | 23 mars 1999 | A. Jakobs |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
............................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**          EP 98 40 3241

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-03-1999

| Document brevet cité au rapport de recherche | Date de publication | Membre(s) de la famille de brevet(s) | | Date de publication |
|---|---|---|---|---|
| FR 2715303      A | 28-07-1995 | FR | 2674753 A | 09-10-1992 |
| | | AU | 1654092 A | 02-11-1992 |
| | | CA | 2107078 A | 03-10-1992 |
| | | EP | 0578733 A | 19-01-1994 |
| | | WO | 9217173 A | 15-10-1992 |
| | | JP | 6506212 T | 14-07-1994 |

EPO FORM P0460

Pour tout renseignement concernant cette annexe : voir Journal Officiel de l'Office européen des brevets, No.12/82